# EUROPÄISCHE PATENTSCHRIFT

(11) **EP 0 693 050 B1**
(45) Veröffentlichungstag und Bekanntmachung des Hinweises auf die Patenterteilung: **01.10.1997**
(21) Anmeldenummer: 94911183.5
(22) Anmeldetag: 16.03.1994
(51) Int. Cl.: C07C 43/13, C07C 41/09, C07C 41/42

(54) **VERFAHREN ZUM HERSTELLEN VON DIGLYCERIN**
PROCESS FOR PREPARING DIGLYCERINE
PROCEDE DE PREPARATION DE DIGLYCERINE

(30) Priorität: 25.03.1993 DE 4309741
(43) Veröffentlichungstag der Anmeldung: 24.01.1996
(73) Patentinhaber: Henkel Kommanditgesellschaft auf Aktien, 40191 Düsseldorf (DE)
(72) Erfinder: JEROMIN, Lutz, D-40723 Hilden (DE); GUTSCHE, Bernhard, D-40721 Hilden (DE); BUNTE, Reinhard, D-41542 Dormagen (DE); JORDAN, Volkmar, D-40822 Mettmann (DE)
(86) Internationale Anmeldenummer: EP9400834
(87) Internationale Veröffentlichungsnummer: WO9421582

(56) Entgegenhaltungen:
- US-A- 2 487 208
- CHEMICAL ABSTRACTS, vol. 113, no. 15, 8. Oktober 1990, Columbus, Ohio, US; abstract no. 132708y, I. NARIBAYASHI ET AL 'PREPARATION OF HIGH-PURITY DIGLYCERIN' Seite 708 ;Spalte 1 ;
- CHEMICAL ABSTRACTS, vol. 98, no. 21, 23. Mai 1983, Columbus, Ohio, US; abstract no. 178739j, 'Diglycerol purification' Seite 600 ;Spalte 1 ;

## Beschreibung

Die Erfindung bezieht sich auf ein Verfahren zum Herstellen von Diglycerin durch Kondensation von Glycerin in Anwesenheit eines basischen Katalysators, vorzugsweise Natrium- oder Kaliumhydroxid, bei Reaktionstemperaturen von 200 bis 275 °C, insbesondere bei Reaktionstemperaturen von 220 bis 240 °C und anschließender Aufkonzentrierung des Diglycerins durch Destillation.

Bei der alkalisch katalysierten Kondensation von Glycerin bei erhöhter Temperatur fallen üblicherweise Diglycerin und höhere Oligomere des Glycerins wie Tri- und Tetraglycerin an, die auch allgemein als Polyglycerine bezeichnet werden. Diese Ether haben relative Molekülmassen von 166 (6 C-Atome) bis 2 238 (90 C-Atome) und 4 bis 32 Hydroxylgruppen. Die Polyglycerine werden neben der basisch katalysierten Dehydratisierung von Glycerin auch durch Reinigung des bei der synthetischen Glycerinherstellung über Epichlorhydrin stammenden polyglycerinhaltigen Destillationsrückstands hergestellt (DE 34 10 520 A1). Die Reinigung wird dabei über einen oder mehrere Kationenaustauscher sowie einen Anionenaustauscher, Wasserverdampfung und Vakuumdestillation vorgenommen.

Zur industriellen Herstellung von Polyglycerin durch Kondensation von Glycerin wird Glycerin zusammen mit einem alkalischen Katalysator bei einer Temperatur zwischen 200 und 275 °C bei Normaldruck oder reduziertem Druck erhitzt. Als Katalysator werden üblicherweise Natriumhydroxid oder Natriumacetat verwendet. In einem typischen Verfahren wird etwa 0,3 % Natriumhydroxid zu Glycerin gegeben und das Reaktionsgemisch auf eine Temperatur von etwa 230 °C aufgeheizt, bei der das Wasser abzudestillieren beginnt. Die Mischung wird dann langsam auf 260 bis 265 °C geheizt und auf dieser Temperatur gehalten, bis die berechnete Menge von Wasser abdestilliert ist. Die für die Entfernung des Wassers erforderliche Zeit ändert sich etwas mit den Reaktionsbedingungen, aber elf Stunden Reaktionszeit zur Herstellung von Diglycerin sind typisch. Das erhaltene Produkt enthält eine Vielzahl von verschiedenen Polyglycerinen sowie nicht umgesetztes Glycerin. Es wird als Diglycerin, Triglycerin, usw. in Abhängigkeit seiner durchschnittlichen Zusammensetzung bezeichnet, die durch die Hydroxylzahl oder die Menge an abdestilliertem Wasser gekennzeichnet ist (Buch: Miner und Dalton, Glycerol, Reinhold Publ. Corp., New York 1953, Seiten 366 bis 368).

Es ist ferner bekannt, die im Reaktionsgemisch vorliegenden Polyglycerine durch Acetylierung und Destillation der Acetate zu trennen. Eine andere Möglichkeit zur Trennung der Polyglycerine besteht in der Destillation ihrer Allyl-Ether oder ihrer Isopropyliden-Derivate. Durch Hydrolyse erhält man dann wieder die Polyglycerine. Ferner ist bekannt, daß die niedrigen Polyglycerine direkt durch Destillation getrennt werden können, wenn ein ausreichend niedriger Druck (Vakuum) angewandt wird.

Eine nachträgliche Auftrennung des Reaktionsgemisches in die einzelnen Polyglycerine ist bei der Herstellung von Diglycerin durch Eigenkondensation von Glycerin nicht möglich. Die nachträgliche Auftrennung entfällt zwar bei einigen bekannten Herstellungsverfahren (G. Jakobson, Fette-Seifen-Anstrichmittel 3(88) 1986, Seiten 101 bis 106), aber diese Herstellungsverfahren haben den Nachteil, daß entweder die Ausgangsubstanzen nur schwer erhältlich sind oder die Synthese über mehrere Zwischenstufen verläuft.

Aus der US 2,487,208 ist ein Verfahren zur Herstellung von Diglycerin durch Kondensation von Glycerin in Gegenwart eines basischen Katalysators bekannt, bei dem man die Reaktion bis zu einem Teilumsatz von 50% des Glycerins durch führt. Die Reaktion wird durch Neutralisation des Katalysators unterbrochen und das Reaktionsgemisch durch Destillation bei einem Druck von 1 mm in Glycerin-, Diglycerin und Restfraktion getrennt.

In einem weiteren bekannten Verfahren zur Herstellung von Polyglycerinen durch Eigenkondensation von Glycerin wird Glycerin in Gegenwart von Lithiumhydroxid bei Temperaturen von 200 bis 300 °C, insbesondere bei 260 °C kondensiert. Das Reaktionswasser wird kontinuierlich destilliert. Wenn ein hoher Glyceringehalt angestrebt wird, so wird die Kondensation abgebrochen, sobald die zur Bildung von Diglycerin theoretisch erforderliche Menge von Wasser abgeschieden worden ist. Das Diglycerin kann dann aus dem gebildeten Oligoglyceringemisch durch Destillation im Hochvakuum abgetrennt werden (DE 41 24 665 A1).

Die Polyglycerine, insbesondere auch Diglycerin, werden als Ausgangsstoffe für Veresterungen bzw. Umesterungen mit Fettsäure bzw. Fettsäureestern eingesetzt. Die Ester der Polyglycerine und auch des Diglycerins bieten eine noch größere Variationsbreite in den Eigenschaften als die einfachen Glyceride und haben daher ein Vielzahl von Anwendungen, von denen nur zwei genannt werden sollen. Sie eignen sich als Rohstoffe zur Herstellung von Polymeren, in dem sie beispielsweise über die Hydroxylgruppen in Alkydharze einkondensiert werden und Polykondensationsbausteine darstellen, wie sie insbesondere für die Entwicklung von Polyurethanschäumen von Wichtigkeit sind (DE 41 24 665 A1). Durch ihre lipophilen und hydrophilen Eigenschaften hat Diglycerin einen weiten Anwendungsbereich als Emulgator in der Kosmetik- und Lebensmittel-Industrie.

Hochreines Diglycerin kann auch durch basenkatalysierte Dimerisierung von Glycerin und anschließende destillative Aufreinigung gemäß Chem. Abstr. vol. 113, 132708y erhalten werden.

Aufgabe der Erfindung ist es, die Herstellung von Diglycerin mit Konzentrationen oberhalb von 90 % auf wirtschaftliche Weise ohne größere Verluste an Ausgangsmaterial zu ermöglichen, wobei als Ausgangsmaterial Glycerin eingesetzt wird, das in Anwesenheit eines basischen Katalysators kondensiert wird.

Diese Aufgabe wird erfindungsgemäß dadurch gelöst, daß man die Reaktion nur bis zu einem Teilumsatz von 10 bis 15 Ma % (die auch im folgenden verwendete Angabe ha% entspricht der ansonst üblicherweise verwendeten Angabe jew.%) Diglycerin im Reaktionsgemisch durchführt und die Reaktion durch Abkühlen des Reaktionsgemisches auf Temperaturen unterhalb von 200 °C unterbricht. Das Reaktionsgemisch wird in einer ersten Destillationsstufe in einem Dünnschichtverdampfer (wiped film evaporater) oder Kurzwegverdampfer bei einem Druck von 0,5 bis 5 mbar, insbesondere bei einem Druck von 1 bis 2 mbar, und bei einer Sumpftemperatur von 125 bis 170 °C, insbesondere bei einer Sumpftemperatur von 130 bis 140 °C destilliert. Als Dünnschichtverdampfer bezeichnet man solche Verdampfer, in denen ein hochviskoses schwersiedendes Gemisch auf eine beheizte Wand aufgegeben und dort durch rotierende Vischelemente mechanisch verteilt wird. Dabei werden dünne geschlossene Flüssigkeitsschichten bzw. Flüssigkeitsfilme erzeugt. Die Filmoberflächen werden ständig erneuert, so daß lokale Überhitzungen vermieden werden. Die entstehenden Dämpfe strömen entgegen dem Fluß des Produktfilms und verlassen den Verdampfer in den außen angeordneten Kondensator. Im Dünnschichtverdampfer wird im allgemeinen bei Drucken von nur einigen mbar gearbeitet, und die Verweildauer für das Produkt beträgt nur wenige Sekunden.

Der genannte Kurzwegverdampfer, der auch als Molekularverdampfer bezeichnet wird, ist zur Destillation noch höher siedender und temperaturempfindlicherer Produkte geeignet. Er ermöglicht durch einen im Verdampfer eingebauten Kondensator Betriebs- bzw. Siededrücke im Bereich des Fein- und Hochvakuums (1 bis 10⁻³ mbar bzw. 10⁻³ bis 10⁻⁵ mbar).

Nach Durchlaufen der ersten Destillationsstufe wird zur Lösung der erfindungsgemäßen Aufgabe der Sumpf aus der ersten Destillationsstufe in einer zweiten, nachfolgenden Destillationsstufe mit einem Kurzwegverdampfer bei einem Druck von 0,05 bis 0,3 mbar, insbesondere bei einem Druck von 0,1 bis 0,2 mbar, und bei einer Sumpftemperatur von 140 bis 170 °C, insbesondere bei einer Sumpftemperatur von 145 bis 155 °C, destilliert, um aus dieser zweiten Destillationsstufe ein Sumpfprodukt mit einem Gehalt von mehr als 90 Ma % Diglycerin zu erhalten.

Die der Erfindung zugrundeliegende Aufgabe wird also in einer aufeinander abgestimmten Kombination von Reaktionsführung und Stofftrennung gelöst. Durch die spezielle Wahl der Reaktionsführung wird die Erzeugung höherer Oligomere des Glycerins, nämlich Tri-, Tetra-, usw. -Glycerin vermieden. Dabei wird bewußt in Kauf genommen, daß das Reaktionsgemisch im Vergleich zu bekannten Verfahren einen relativ niedrigen Gehalt an Diglycerin aufweist. Dieser Nachteil wird durch die nachgeschalteten beiden Destillations stufen wieder ausgeglichen, so daß als Endprodukt ein Diglycerin mit einem Gehalt von mehr als 90 % erhalten wird. Die Probleme der bekannten Verfahren, daß zur Erhöhung des Diglycerin-Anteils sowohl Glycerin als auch höhere Oligomere des Glycerin abgetrennt werden müssen und daß außerdem die Entstehung dieser höheren Oligomere die Wirtschaftlichkeit des Verfahrens beeinträchtigt, treten im erfindungsgemäßen Herstellungsverfahren nicht mehr auf, da schon das Entstehen der höheren Oligomere vermieden wird.

Das Verfahren läßt sich sowohl diskontinuierlich als auch kontinuierlich durchführen. In einer bevorzugten Ausführungsform der Erfindung führt man die Reaktion kontinuierlich im Rohreaktor durch. Der Katalysator (z. B. NaOH) wird als Lösung zudosiert.

Bei der kontinuierlichen Fahrweise ist es vorteilhaft, wenn im Reaktor möglichst keine Rückvermischung auftritt. Daher wird, insbesondere bei größeren Durchmessern, z. B. bei Durchmessern von mehr als 0,3 m, vorgeschlagen, daß der Rohrreaktor inerte Füllkörper zur Unterdrückung von Zirkulationsströmungen enthält. Alternativ zum homogen gelösten Katalysator kann auch ein Katalysator enthaltendes Festbett eingesetzt werden. Bei dem Festbett-Katalysator kann es sich z. B. um einen NaX-Zeolith, insbesondere Wessalith P (Produkt der Fa. Henkel KGaA), handeln. Ferner ist es bei der kontinuierlichen Fahrweise vorteilhaft, wenn man nach der Reaktion das entstandene Reaktionsgemisch trocknet, also das Reaktionswasser abtrennt, bevor man das Reaktionsgemisch der ersten Destillationsstufe unterwirft. Bei der diskontinuierlichen Fahrweise wird ein Großteil des Reaktionswassers bereits während der Reaktion abdestilliert.

Es ist auch möglich, das erfindungsgemäße Verfahren diskontinuierlich durchzuführen, in dem der Rohrreaktor durch einen Rührkessel ersetzt wird.

Charakteristisch für das Verfahren ist, daß im wesentlichen nur Diglycerin, Reaktionswasser und nicht umgesetztes Glycerin anfällt. Vorteilhaft ist es, daß man das Destillat der ersten Destillationsstufe, das aus nahezu reinem Glycerin besteht, in die Reaktionsstufe zurückführt. Es ist aber auch möglich, dieses Destillat als Ausgangssubstanz in anderen Verfahren zu verwenden. Die Rückführung in die Reaktionsstufe ist allerdings besonders vorteilhaft, da in diesem Fall in dem gesamten Verfahren hauptsächlich nur Reaktionswasser und reines Diglycerin anfällt. Nur geringe Produktverluste treten auf.

Zur weiteren Verringerung der Produktverluste ist es vorteilhaft, daß man das Destillat der zweiten Destillationsstufe, das Glycerin und Diglycerin enthält, dem Zulauf der ersten Destillationsstufe zumischt und damit in diese Stufe zurückführt.

Der Sumpf der zweiten Destillationsstufe hat einen Diglyceringehalt von mehr als 90 % und einen verschwindend geringen Glyceringehalt. Die Reinheit des Produktes läßt sich in einer weiteren vorteilhaften Ausführungsform des erfindungsgemäßen Verfahrens steigern, indem man das Sumpfprodukt der zweiten Destillationsstufe in einem Kurzwegverdampfer bei einem Druck von höchstens 0,05 mbar und einer Temperatur von 185 bis 215 °C in einer dritten Destillationsstufe destilliert, um ein Destillat mit einem Gehalt von mehr als 95 Ma % Diglycerin zu erhalten. Diese Feindestillation erhöht nicht nur den Gehalt an Diglycerin, sondern entsalzt und entfärbt außerdem das Produkt.

Im nachfolgenden werden Ausführungsbeispiele der Erfindung beschrieben.

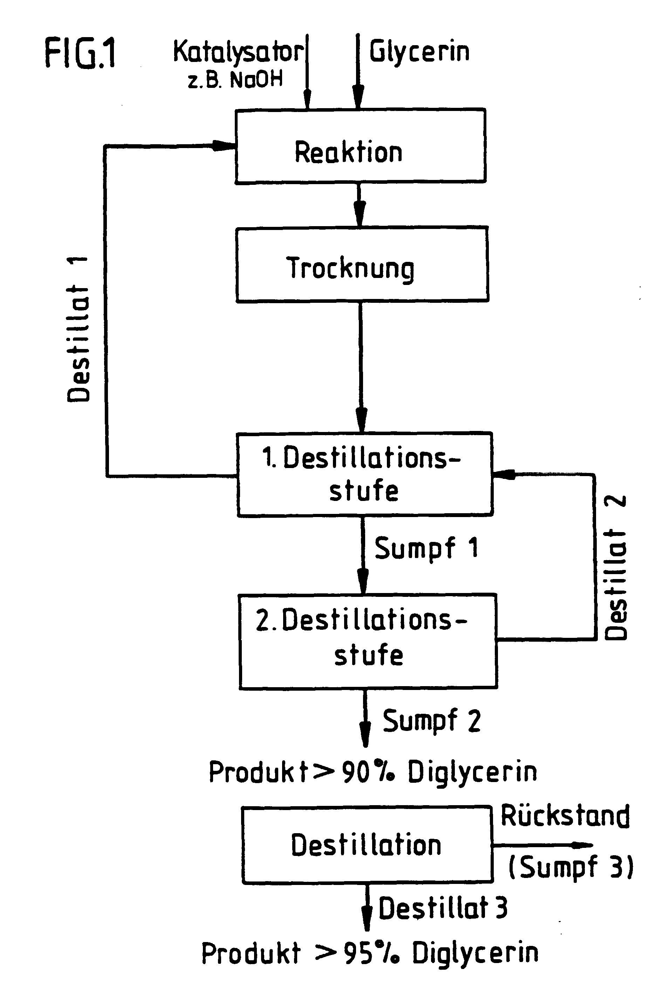

Die Erfindung mit ihren wesentlichen Merkmalen und wichtigsten vorteilhaften Ausgestaltungen ist zusammengefaßt in Figur 1 in einem Fließbild dargestellt. Nach Zugabe von Katalysator (Natriumhydroxid) und Glycerin wird die Kondensationsreaktion bis zum Teilumsatz von 10 bis 15 Ma % durchgeführt. Die Reaktion wird durch Abkühlen unterbrochen, und das Reaktionsgemisch wird im Fall des kontinuierlich durchgeführten Verfahrens getrocknet. Das in der darauffolgenden ersten Destillationsstufe erhaltene, nahezu ausschließlich aus Glycerin bestehende Destillat 1 wird in die Reaktionsstufe zurückgeführt. Der erhaltene Sumpf 1 wird in einer zweiten Destillationsstufe in ein Destillat 2 und einen Sumpf 2 aufgetrennt. Das Destillat 2 wird in die erste Destillationsstufe zurückgeführt. Der Sumpf 2 enthält mehr als 90 % Diglycerin und ist in vielen Fällen das erwünschte Endprodukt. In anderen Fällen, wenn ein noch höherer Diglyceringehalt gefordert ist, wird der Sumpf 2 in einer dritten Destillationsstufe zu einem Destillat 3 mit mehr als 95 % Diglycerin sowie einem Rückstand (Sumpf 3) aufgetrennt.

### Beispiel 1 (Diskontinuierliche Reaktionsführung)

Glycerin wurde mit dem Katalysator NaOH (Einwaage 0,5 Ma % der Glycerinmenge) bei einer Temperatur von 230 °C im Rührreaktor umgesetzt.

Nach 8 Stunden Reaktionszeit ergibt sich ein Diglyceringehalt von 12,5 Ma %. Der Gehalt an Triglycerin beträgt 1 %. Der Anteil höherer Oligoglycerine liegt unterhalb der Nachweisgrenze von 0,1 Ma %.

### Beispiel 2 (Kontinuierliche Reaktionsführung)

Glycerin wurde mit dem Katalysator NaOH (Dosierung 0,35 Ma % bezogen auf die Glycerinmasse) bei einer Temperatur von 240 °C im Rohrreaktor, der zur Unterdrückung von Rückvermischungseffekten mit inerten Füllkörpern gefüllt war, umgesetzt.

Es ergab sich ein Diglyceringehalt von 12 Ma %, der Gehalt an Triglycerin war 0,8 Ma %, höhere Oligoglycerine waren nicht nachweisbar (< 0,1 Ma %).

### Beispiel 3 (Kontinuierliche Reaktionsführung, heterogen katalysiert)

Glycerin wurde bei 240 °C im Festbettreaktor, der mit einem NaX-Katalysator (Zeolith vom Typ Wessalith P) gefüllt war, umgesetzt. Es ergab sich ein Diglyceringehalt von 12,5 % und ein Gehalt an höheren Glycerinen von 0,9 %.

### Beispiel 4 (Zweistufige Destillation)

Das Reaktionsgemisch aus Beispiel 1 wurde zweistufig destilliert.

Die erste Stufe der Destillation wurde im Kurzwegverdampfer bei einem Druck von 1,2 mbar und einer Temperatur von 140 °C durchgeführt. Die zweite Stufe der Destillation wurde ebenfalls im Kurzwegverdampfer, jedoch bei einem Druck von 0,1 mbar und einer Temperatur von 155 °C durchgeführt. Das Destillat der zweiten Stufe wurde kontinuierlich in die erste Destillationsstufe zurückgeführt. Es ergaben sich folgende Konzentrationen:

| | Destillat | Sumpf |
|---|---|---|
| 1. Stufe | < 0,5 Ma % Diglycerin | 30 - 40 Ma % Diglycerin |
| 2. Stufe | 15 Ma % Diglycerin | 90 - 91,5 Ma % Diglycerin |

### Beispiel 5 (3. Destillationsstufe)

Das Produkt der Destillation (Sumpf der 2. Stufe) aus Beispiel 4 wurde bei einer Temperatur von 205 °C und einem Druck von 10⁻² mbar im Kurzwegverdampfer destilliert. Das Destillat war 98 %-iges salzfreies Diglycerin.

Der Verlust (Sumpf der 3. Stufe) in Form von Rückstand (Salz und Polyglycerin) betrug 25 Ma %.

## Patentansprüche

1. Verfahren zum Herstellen von Diglycerin durch Kondensation von Glycerin in Anwesenheit eines basischen Katalysators, vorzugsweise Natrium- oder Kaliumhydroxid, bei Reaktionstemperaturen von 200 bis 275 °C, insbesondere bei Reaktionstemperaturen von 220 bis 240 °C und anschließender Aufkonzentrierung des Diglycerins durch Destillation,
dadurch gekennzeichnet,
daß man die Reaktion nur bis zu einem Teilumsatz von 10 bis 15 Ma % Diglycerin im Reaktionsgemisch durchführt, die Reaktion durch Abkühlen des Reaktionsgemisches auf Temperaturen unterhalb von 200 °C unterbricht, das Reaktionsgemisch in einer ersten Destillationsstufe in einem Dünnschichtverdampfer (wiped film evaporater) oder Kurzwegverdampfer bei einem Druck von 0,5 bis 5 mbar, insbesondere bei einem Druck von 1 bis 2 mbar, und bei einer Sumpftemperatur von 125 bis 170 °C, insbesondere bei einer Sumpftemperatur von 130 bis 140 °C destilliert und in einer zweiten, nachfolgenden Destillationsstufe den Sumpf aus der ersten Destillationsstufe mit einem Kurzwegverdampfer bei einem Druck von 0,05 bis 0,3 mbar, insbesondere bei einem Druck von 0,1 bis 0,2 mbar, und bei einer Sumpftemperatur von 140 bis 170 °C, insbesondere bei einer Sumpftemperatur von 145 bis 155 °C, destilliert, um aus dieser zweiten Destillationsstufe ein Sumpfprodukt mit einem Gehalt von mehr als 90 Ma % Diglycerin zu erhalten.

2. Verfahren nach Anspruch 1, wobei man die Reaktion kontinuierlich im Rohrreaktor durchführt, der ein den Katalysator enthaltendes Festbett aufweist.

3. Verfahren nach Anspruch 2, wobei der Rohrreaktor inerte Füllkörper enthält.

4. Verfahren nach Anspruch 2 oder 3, wobei man nach der Reaktion das entstandene Reaktionsgemisch trocknet, bevor man es der ersten Destillationsstufe unterwirft.

5. Verfahren nach Anspruch 1, wobei man die Reaktion diskontinuierlich im Rührkessel durchführt.

6. Verfahren nach einem der Ansprüche 1 bis 5, wobei man das Destillat der ersten Destillationsstufe in die Reaktionsstufe zurückführt.

7. Verfahren nach einem der Ansprüche 1 bis 6, wobei man das Destillat der zweiten Destillationsstufe in die erste Destillationsstufe zurückführt.

8. Verfahren nach einem der Ansprüche 1 bis 7, wobei man das Sumpfprodukt der zweiten Destillationsstufe in einem Kurzwegverdampfer bei einem Druck von höchstens 0,05 mbar und einer Temperatur von 185 bis 215 °C in einer dritten Destillationsstufe destilliert, um ein Destillat mit einem Gehalt von mehr als 95 Ma % Diglycerin zu erhalten.

## Claims

1. A process for the production of diglycerol by condensation of glycerol in the presence of a basic catalyst, preferably sodium or potassium hydroxide, at reaction temperatures of 200 to 275°C and, more particularly, at reaction temperatures of 220 to 240°C and subsequent concentration of the diglycerol by distillation, characterized in that the reaction is only carried out to a partial conversion of 10 to 15% by weight of diglycerol in the reaction mixture and is terminated by cooling the reaction mixture to temperatures below 200°C; in a first distillation stage, the reaction mixture is distilled in a wiped-film evaporator or short-path evaporator under a pressure of 0.5 to 5 mbar, more particularly under a pressure of 1 to 2 mbar, and at a bottom temperature of 125 to 170°C, more particularly at a bottom temperature of 130 to 140.C; the sump from the first distillation stage is distilled in a second, following distillation stage carried out in a short path evaporator under a pressure of 0.05 to 0.3 mbar and, more particularly, under a pressure of 0.1 to 0.2 mbar and at a bottom temperature of 140 to 170°C and, more particularly, at a bottom temperature of 145 to 155°C in order to obtain a bottom product containing more than 90% by weight of diglycerol in this second distillation stage.

2. A process as claimed in claim 1, characterized in that the reaction is carried out continuously in a tube reactor containing a fixed bed of catalyst.

3. A process as claimed in claim 2, characterized in that the tube reactor contains inert packing elements.

4. A process as claimed in claim 2 or 3, characterized in that, after the reaction, the reaction mixture is dried before being subjected to the first distillation stage.

5. A process as claimed in claim 1, characterized in that the reaction is carried out discontinuously in a stirred tank reactor.

6. A process as claimed in any of claims 1 to 5, characterized in that the distillate of the first distillation stage is returned to the reaction stage.

7. A process as claimed in any of claims 1 to 6, characterized in that the distillate of the second distillation stage is returned to the first distillation stage.

8. A process as claimed in any of claims 1 to 7, characterized in that the sump product of the second distillation stage is distilled in a third distillation stage carried out in a short-path evaporator under a pressure of at most 0.05 mbar and at a temperature of 185 to 215°C in order to obtain a distillate containing more than 95% by weight of diglycerol.

## Revendications

1. Procédé pour la préparation de diglycérine par condensation de glycérol en présence d'un catalyseur basique, de préférence l'hydroxyde de sodium ou de potassium, à des températures réactionnelles de 200 à 275°C, en particulier à des températures réactionnelles de 220 à 240°C, et par surconcentration ultérieure de la diglycérine par distillation, caractérisé en ce que
on effectue la réaction seulement jusqu'à une conversion partielle de la diglycérine à concurrence de 10 à 15 Ma % dans le mélange réactionnel, on interrompt la réaction par refroidissement du mélange réactionnel à des températures inférieures à 200°C, on distille le mélange réactionnel dans une première étape de distillation dans un évaporateur en couche mince (wiped film evaporater) ou dans un évaporateur moléculaire sous une pression de 0,5 à 5 mbar, en particulier sous une pression de 1 à 2 mbar et à une température du produit de bas de colonne de 125 à 170°C, en particulier à une température du produit de bas de colonne de 130 à 140°C et, dans une deuxième étape de distillation ultérieure, on distille le produit de bas de colonne provenant de la première étape de distillation à l'aide d'un évaporateur moléculaire sous une pression de 0,05 à 0,3 mbar, en particulier sous une pression de 0,1 à 0,2 mbar et à une température du produit de bas de colonne de 140 à 170°C, en particulier à une température du produit de bas de colonne de 145 à 155°C pour obtenir, à partir de cette deuxième étape de distillation, un produit de bas de colonne dont la teneur en diglycérine est supérieure à 90 Ma %.

2. Procédé selon la revendication 1, dans lequel on effectue la réaction en continu dans un réacteur tubulaire qui présente un lit fixe contenant le catalyseur.

3. Procédé selon la revendication 2, dans lequel le réacteur tubulaire contient des corps de remplissage inertes.

4. Procédé selon la revendication 2 ou 3, dans lequel on sèche le mélange réactionnel obtenu après la mise en réaction avant de le soumettre à la première étape de distillation.

5. Procédé selon la revendication 1, caractérisé en ce qu'on effectue la réaction en discontinu dans une cuve d'agitation.

6. Procédé selon l'une quelconque des revendications 1 à 5, dans lequel on recycle dans l'étape réactionnelle le distillat de la première étape de distillation.

7. Procédé selon l'une quelconque des revendications 1 à 6, dans lequel on recycle dans la première étape de distillation le distillat de la deuxième étape de distillation.

8. Procédé selon l'une quelconque des revendications 1 à 7, dans lequel on distille le produit de bas de colonne de la deuxième étape de distillation dans un évaporateur moléculaire sous une pression de 0,05 mbar au maximum et à une température de 185 à 215°C, dans une troisième étape de distillation, pour obtenir un distillat dont la teneur en diglycérine est supérieure à 95 Ma %.
